# EUROPÄISCHE PATENTANMELDUNG

(11) **EP 0 906 748 A2**
(43) Veröffentlichungstag der Anmeldung: **07.04.1999**
(21) Anmeldenummer: 98122557.6
(22) Anmeldetag: 09.03.1994
(51) Int. Cl.: A61B 17/22

(54) **Medizinisches Instrument für Atherektomie**

(30) Priorität: 11.03.1993 DE 4307642
(62) Teilanmeldung aus: 94911137.1
(71) Anmelder: Redha, Falah, Dr., 8606 Greifensee (CH)
(72) Erfinder: Redha, Falah, Dr., 8606 Greifensee (CH)
(74) Vertreter: Jeck, Anton, Dipl.-Ing.

(57) **Zusammenfassung**

Die Erfindung betrifft ein medizinisches Instrument zur Beseitigung von Ablagerungen in Arterien- und/oder Venenwänden mit einem in die Gefäße einbringbaren und zumindest teilweise hohlen Körper (10-14) mit einem aus den Gefäßen ausführbaren Zug- und/oder Betätigungsmittel (20) sowie mit in Zugrichtung weisenden und die Ablagerungen beseitigenden Schneidekörpern (21, 22) mit Schneidekanten (24). In den Körper (10, 14) sind zumindest teilweise hineinragende Grundkörper (26, 27) an dem die in Zugrichtung sich erstreckenden und als angeformte Blättchenfeder ausgebildete Schneidekörper (21, 22) angelenkt.

## Beschreibung

Die Erfindung betrifft ein medizinisches Instrument nach dem Oberbegriff des Anspruches 1.

Bei herkömmlichen Instrumenten der eingangs genannten Art, wie sie z. B. in der US-PS 4765 332 beschrieben sind, ist ein etwa zylinderförmiger Grundkörper mit in Zugrichtung weisenden Schneideabschnitten vorgesehen, die ebenfalls zylinderförmig sind. Die scharfen Kanten der Schneideabschnitte verjüngen sich von innen nach aussen, was zur Verletzung auch gesunder Gefäße führen kann. Ein weiterer Nachteil, mit dem das bekannte Instrument behaftet ist, besteht darin, dass eine Anpassung an die Arterien- bzw. Venenwände nur im begrenzten Umfang möglich ist.

Schließlich ist in der WO-A-9002523 ein medizinisches Instrument, gemäß dem Oberbegriff des Anspruchs 1, offenbart, bei dem die schalenförmigen Teile (Schneidekörper) mit einem Spannkörper zusammenarbeiten. Durch diesen Spannkörper ist der Abstand der Schneidekanten voneinander zwar einstellbar, die Handhabung mit dem Instrument ist jedoch umständlich.

Ausgehend vom obigen Stand der Technik liegt der Erfindung die Aufgabe zugrunde, das gattungsgemäße Instrument so weiter zu bilden, dass sein effektiver Querschnitt einfach vergrößert und den Venen angepasst werden kann.

Die gestelte Aufgabe wird erfindungsgemäß durch die Merkmale des Anspruches 1 gelöst.

Ein solches Instrument kann in das Gefäß mittels eines Katheters eingeführt werden, und zwar bis zur vorgesehenen Stelle, die durch Kontrastmittel lokalisiert werden kann. Der Katheter und der Körper werden so angeordnet, dass die Plaque zwischen dem Körper und dem Katheter angeordnet ist. Dann wird das Zugmittel betätigt und die vorgesehene Stelle wird abgeschabt. Dieser Vorgang kann so oft wiederholt werden, bis eine glatte Innenwand vorhanden ist. Die beim Hoben entstandenen Späne können entweder abgesaugt oder durch den Körper, der nach dem Eingriff aus der Arterie herausgezogen wird, mitgenommen werden.

Weitere zweckmäßige und vorteilhafte Ausgestaltungen der Erfindung gehen aus den Unteransprüchen hervor.

Eine zweckmäßige Ausgestaltung der Erfindung sieht vor, dass der in den Körper hineinragende Abschnitt des Grundkörpers als ein Rohrabschnitt ausgebildet ist, in dem ein Organ zur relativen Verstellung des Körpers mit Bezug auf den Schneidekörper angeordnet ist.

Dabei ist es zweckmäßig, wenn das Organ als ein Rohrstück oder Drahtstück ausgeführt ist, das von aussen her betätigbar ist. Durch die Betätigung des Organs wird auf die Schneidekörper Druck ausgeübt, so dass sie den effektiven Querschnitt des Schneidekörpers entweder vergrößern oder verkleinern, und zwar abhängig davon, ob der Körper zum Benutzer hin oder von diesem weg bewegt wird.

Eine weitere zweckmäßige Ausgestaltung sieht vor, dass die Schwenkachse der Schneidekörper sich quer zur Zugrichtung des Körpers erstreckt. Dadurch wird sichergestellt, dass die Schneidekanten zum Benutzer hin weisen und die Schneidekörper ihre wirksame bzw. unwirksame Position einnehmen können. Gemäß dem Erfindungsgedanken werden die Schneidekörper als zweiarmige Hebel ausgebildet, wobei die zwei Schneidkörper eine gemeinsame Schwenkachse und jeweils einen Kraft- und einen Lastarm aufweisen. Die Schneidekörper können in ihrer Ruhestellung vollständig oder annähernd vollständig im Körper untergebracht werden. Dabei kann der Grundkörper einen Anschlagabschnitt aufweisen, der in Ruhestellung der Schneidekörper annähernd abstandsfrei zu einer Stirnseite des Körpers angeordnet ist. Der Vorteil dieser Maßnahme besteht insbesondere darin, dass das Einbringen des Instruments in Gefäße deutlich erleichtert wird, da in diesem Fall die Schneidekanten mit dem Gefäß nicht in Berührung kommen.

Die Innenwand des Körpers kann Druckkörper aufweisen, die in ihrer Betriebsstellung mit dem jeweiligen Kraftarm und in ihrer Ruhestellung mit dem jeweiligen Lastarm der Schneidekörper in Druckverbindung stehen. Die Druckkörper können als radiale Vorsprünge mit Gleitkurven ausgebildet sein. Der Vorteil dieser Maßnahmen besteht darin, dass durch axiale Versetzung des Körpers in Zugrichtung die Schneidekörper entweder geöffnet oder geschlossen werden. Beim Öffnen fahren die Schneidekörper aus dem Körper heraus, dabei wird auf den jeweiligen Kraftarm des Schneidekörpers seitens des Körpers Druck ausgeübt, so dass sich die Schneidekörper öffnen und außerhalb des Körpers angeordnet sind. Die Schneidekanten der Schneidekörper definieren hierbei einen Kreis oder eine Ellipse, der bzw. die außerhalb des Körpers angeordnet ist/sind. Um die Schneidekörper kontinuierlich betätigen zu können, ist vorgesehen, dass das Außenprofil des jeweiligen Schneidekörper-Längsschnittes bogenförmig ist. Durch diese Maßnahmen wird ein allmähliches Öffnen bzw. Schließen der Schneidekörper sichergestellt.

Ferner ist vorgesehen, dass der Körper im Wesentlichen als ein Hohlzylinder ausgebildet ist, der die Schneidekörper in ihrer Ruhestellung aufnimmt.

Eine weitere zweckmäßige Ausgestaltung der Erfindung sieht vor, dass die vom Benutzer abgekehrte Stirnseite der Körper eine in Zugrichtung sich erstreckende Öffnung aufweist. Die Öffnung kann zur Aufnahme eines Führungsmittels, z. B. eines dünnen Drahtstückes, dienen, durch das die Einbringung des Instruments an die vorgesehene Stelle erleichtert werden kann. Dabei ist es zweckmäßig, wenn die vom Benutzer abgekehrte Stirnseite des Körpers konvex und kantfrei ist.

Eine weitere zweckmäßige Ausgestaltung sieht vor, dass das als Seil-, Rohr- oder Drahtstück ausgebildete Organ Klemmkörper trägt, die abhängig von der Betriebslage der Schneidekörper mit dem Kraft- bzw. Lastarm des jeweiligen Schneidekörpers zusammenarbeiten. Dabei können die Klemmkörper im axialen Querschnitt keilförmig ausgebildet und mit ihren verjüngten Stirnseiten einander zugekehrt sein. Ferner ist es besonders zweckmäßig, wenn zwei Klemmkörper vorgesehen sind, zwischen denen die Schwenkachse der Schneidekörper angeordnet ist. Einer der Klemmkörper kann hierbei vom Grundkörper getragen sein und aus einem elastisch verformbaren Werkstoff bestehen. In diesem Fall übt nur einer der Klemmkörper Relativbewegungen mit Bezug auf die Schwenkachse der Schneidekörper aus.

Eine vorteilhafte Ausgestaltung der Erfindung sieht vor, dass die an die Schneidekanten sich anschließenden Flanken der Schneidekörper zumindest teilweise mit Schneideabschnitten versehen sind. Durch diese Maßnahmen ist sichergestellt, dass beim Schließen der Schneidekörper nicht nur die stirnseitigen Schneidekanten, sondern auch die in Längsrichtung der Schneidekörper sich erstreckenden Kanten als Schneideorgane wirken.

Schließlich sieht eine besonders zweckmäßige Ausgestaltung der Erfindung vor, dass mehrere Schneidekörper-Paare in Serie geschaltet sind, wodurch die Beseitigung der Ablagerungen schneller erfolgen kann.

Einige Ausführungsbeispiele der Erfindung sind in der Zeichnung schematisch dargestellt und werden im Folgenden näher erläutert.

Es zeigen
- Fig. 1: einen axialen Querschnitt eines Teiles der ersten Ausführungsform des Instruments in Betriebsstellung,
- Fig. 2: das in Fig. 1 dargestellte Instrument in Ruhestellung,
- Fig. 3: eine zweite Ausführungsform des Instrumentes in Betriebsstellung,
- Fig. 4: das in Fig. 3 dargestellte Instrument in Ruhestellung,
- Fig. 5: einen Schnitt entlang der Linie A-A,
- Fig. 6: eine weitere Ausführungsform des Instrumentes in seiner Betriebsstellung,
- Fig. 7: eine weitere Ausführungsform des Instrumentes in seiner Schließstellung,
- Fig. 8: ein Instrument mit Druckkörpern in Betriebsstellung,
- Fig. 9: das in Fig. 8 dargestellte Instrument in Ruhestellung,
- Fig. 10: ein Instrument mit Klemmkörpern und einer Querbohrung in Betriebsstellung,
- Fig. 11: ein Instrument mit elastischen Klemmkörpern in Betriebsstellung und
- Fig. 12: das Instrument nach Fig. 1 in Tandem-Ausführung.

In den Figuren sind medizinische Instrumente zur Beseitigung von Ablagerungen in Arterien- und/oder Venenwänden mit jeweils einem in die Gefäße einbringbaren und zumindest teilweise hohlen Körper 10 - 14 mit einem aus den Gefäßen ausführbaren Zug- und/oder Betätigungsmittel 20 sowie mit in Zugrichtung weisenden und die Ablagerungen beseitigenden Schneidekörpern 21, 22 mit Schneidekanten 24 dargestellt. Dabei ist ein in den Körper 10 - 14 zumindest teilweise hineinragender Grundkörper 26, 27 vorgesehen, an dem die in Zugrichtung sich erstreckenden Schneidekörper 21, 22 angelenkt sind und ihre Betriebs- bzw. Ruhestellung durch Verstellung des Körpers 10 - 14 in Zugrichtung definierbar ist. Der in den jeweiligen Körper 10 - 14 hineinragende Abschnitt 30 des Grundkörpers 26, 27 ist als ein Rohrabschnitt ausgebildet, in dem ein Organ 32, 34 zur relativen Verstellung des Körpers 10 - 14 mit Bezug auf den Schneidekörper 21, 22 angeordnet ist. Das Organ 32, 34 ist als ein Rohrstück oder Drahtstück ausgeführt, das von aussen her betätigbar ist. Die Schwenkachse 36 der Schneidekörper 21, 22 erstreckt sich quer zur Zugrichtung des Körpers 10 - 14.

Man erkennt, dass die Schneidekörper 21 und 22 als zweiarmige Hebel ausgebildet sind, die in ihren Ruhestellungen vollständig oder annähernd vollständig im Körper 10 - 14 untergebracht sind. Der Grundkörper 26 bzw. 27 weist einen Anschlagabschnitt 38 auf, der in Ruhestellung der Schneidekörper 21 und 22 annähernd abstandsfrei zu einer Stirnseite 40 des jeweiligen Körpers 10 - 14 angeordnet ist. Der jeweilige Körper 10 - 14 ist im Wesentlichen als ein Hohlzylinder ausgebildet, der die Schneidekörper 21 und 22 in ihrer Ruhestellung aufnimmt. Die vom Benutzer abgekehrte Stirnseite 41 des jeweiligen Körpers 10 - 14 weist eine in Zugrichtung sich erstreckende Öffnung 42 auf, in die das Endstück des Organs 32 bzw. 34 hereinragt und dort befestigt ist. Die vom Benutzer abgekehrte Stirnseite 41 des jeweiligen Körpers 10 - 14 ist konvex und kantfrei.

Die Figuren lassen ferner erkennen, dass zwei Schneidekörper 21, 22 mit einer gemeinsamen Schwenkachse 36 und jeweils einem Kraft- und einem Lastarm 43 und 44 vorgesehen sind. Dabei weist die Innenwand des jeweiligen Körpers 10, 11, 12 Druckkörper 46 auf, die in ihrer Betriebsstellung mit dem jeweiligen Kraftarm 43 und in ihrer Ruhestellung mit dem jeweiligen Lastarm 44 der Schneidekörper 21 und 22 in Druckverbindung stehen. Die Druckkörper 46 sind hierbei als radiale Vorsprünge mit Gleitkurven, die sich in axialer Richtung erstrecken, ausgebildet. Der Aussenumriss des jeweiligen Schneidekörper-Längsschnittes ist bogenförmig, so dass ein kontinuierliches Öffnen bzw. Schließen der Schneidekörper gewährleistet ist.

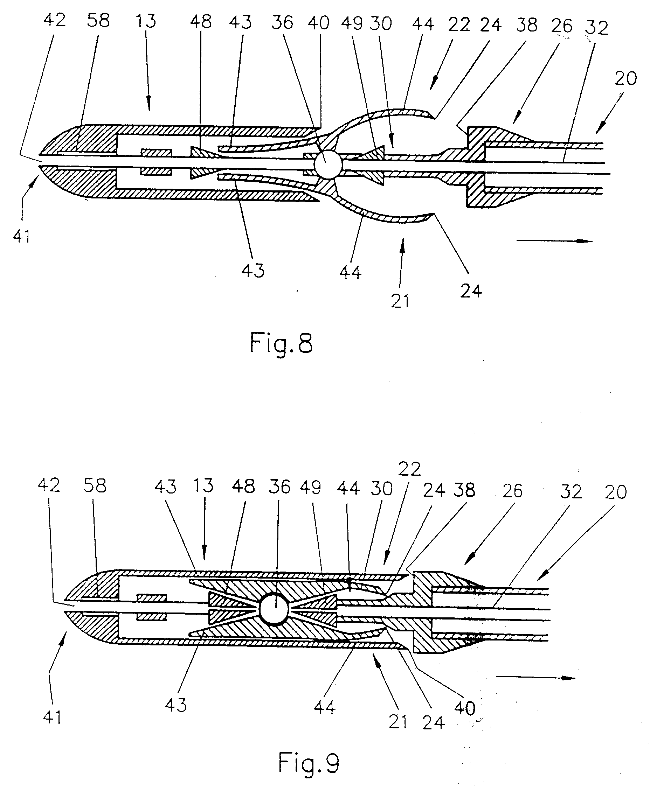

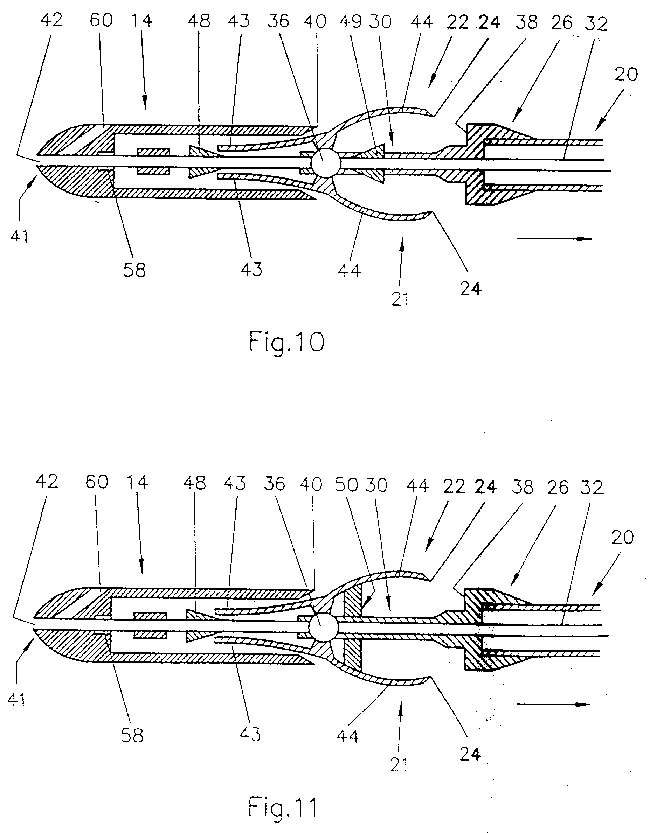

Das als Seil-, Rohr- oder Drahtstück ausgebildete Organ 32, 34 trägt Klemmkörper 48, 49 und 50 (vgl. Fig. 8 - 11), die abhängig von der Betriebslage der Schneidekörper 21 und 22 mit dem Kraft- bzw. Lastarm 43 und 44 des jeweiligen Schneidekörpers 21 und 22 zusammenarbeiten. Die Klemmkörper 48, 49 und 50 sind im axialen Querschnitt keilförmig ausgebildet und mit ihren verjüngten Stirnseiten einander zugekehrt. Hierbei sind jeweils zwei Klemmkörper 48, 49 und 50 vorgesehen, zwischen denen die Schwenkachse der Schneidekörper 21 und 22 angeordnet ist. Der in Fig. 11 dargestellte Klemmkörper 50 ist vom Grundkörper 26 getragen und besteht aus einem elastisch verformbaren Werkstoff.

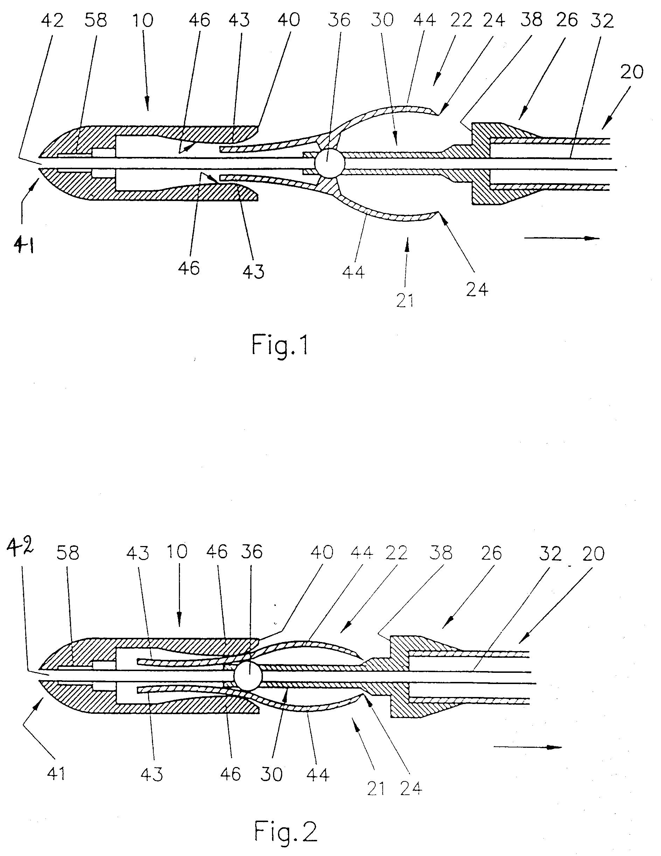

Das in Fig. 1 und 2 dargestellte Teil des medizinischen Instruments weist ferner die Besonderheit auf, dass der Körper 10 die Schneidekörper 21 und 22 in ihrer Ruhestellung (Fig. 2) nicht vollständig aufnimmt. Die Schneidekanten 24 sind jedoch vor dem Anschlag 38 angeordnet, so dass es in diesem Fall zu keinem Schneidevorgang kommen kann. Der Schneidevorgang wird erst dann gewährleistet, wenn der Körper 10 einen etwa maximalen Abstand von der Schwenkachse 36 aufweist, wobei seine Druckkörper 46 auf die Kraftarme 43 Druck ausüben, so dass die Schneidekanten 24 maximal voneinander angeordnet sind und den Grundkörper 26 überragen, d. h. der Aussenumfang des Grundkörpers 26 ist hierbei kleiner als der Aussenumfang der beiden Schneidekanten 24. Da die Schneidekörper 21 und 22 am Abschnitt 30, der als Rohrstück ausgebildet ist, angelenkt sind, wird beim Verschieben des Organs 32, das ebenfalls als ein Rohrstück ausgebildet ist, der Körper 10 wegbewegt, wobei auf die Kraftarme 43 Kraft ausgeübt wird. In das Organ 32 kann ein Führungskörper, z. B. Drahtstück, eingebracht werden, das aus der Öffnung 42 herausragt und zur Einführung des Instrumentes in die Gefäße dienen kann. Wird das Organ 32 in Richtung des Pfeiles betätigt, dann wird der Abstand des Körpers 10 von der Schwenkachse 36 minimiert, wobei die Druckkörper 46 auf die Lastarme 44 Druck ausüben, so dass das Instrument die in Fig. 2 dargestellte Position einnimmt.

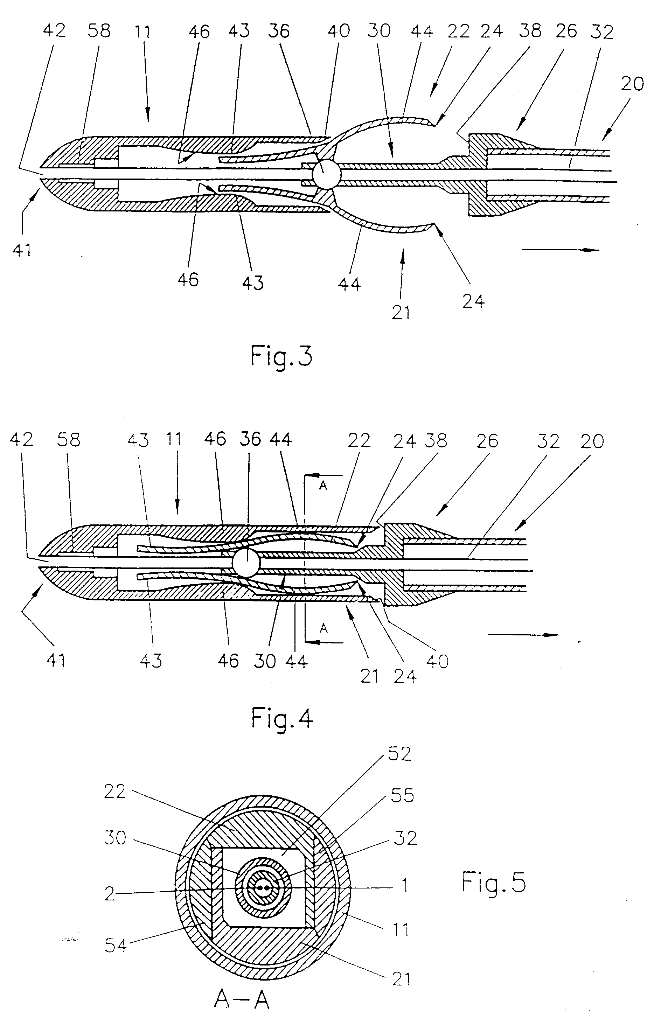

In den Figuren 3 bis 5 ist ein Teil eines weiteren Instruments dargestellt, das sich von dem Instrument nach Figuren 1 und 2 dadurch unterscheidet, dass der Körper 11 so bemessen ist, dass er in Schließstellung bzw. Ruhestellung des Instruments beide Schneidekörper 21 und 22 voll aufnehmen kann (vgl. Fig. 4). Der Aussenumriss des Querschnittes des Körpers 11 ist kreisrund. Den gleichen Querschnitt hat auch der Grundkörper 26, so dass der Körper 11 mit dem Grundkörper 26 bündig abschließen kann. Ein Vorteil dieser Maßnahme besteht darin, dass das Einbringen des Instruments in die Gefäße besonders einfach ist.

Die Fig. 5 lässt erkennen, dass auch die in Zugrichtung des Instruments sich erstreckenden Flanken 54 und 55 als Schneidekörper ausgebildet sind, so dass nicht nur die Stirnseite, sondern auch die Längsseiten zum Schneiden dienen können. Das Organ 32 ist hierbei so bemessen, dass es gleichzeitig auch zur Führung eines Angioscopen 3 und eines Führungskörpers 1 dienen kann.

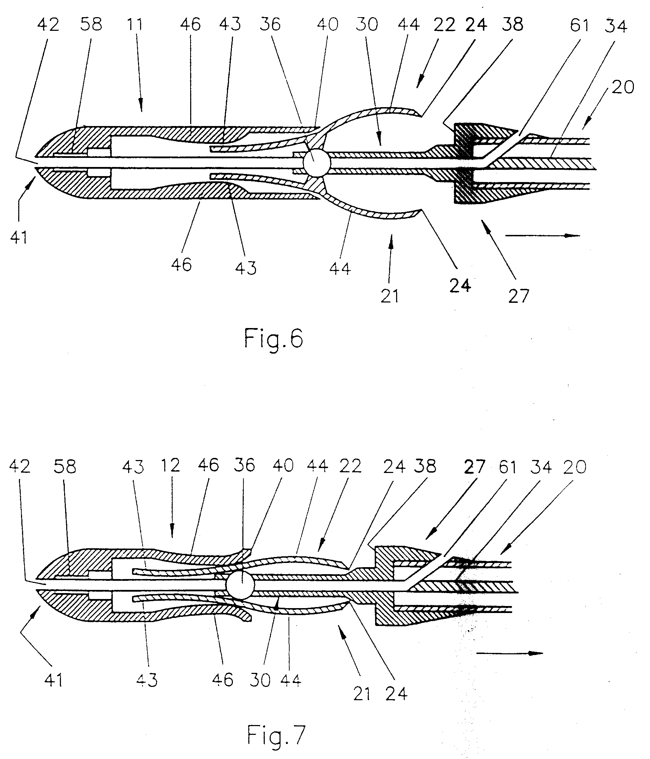

Die Besonderheit des Instruments, wie es in Fig. 7 dargestellt ist, besteht darin, dass der Körper 12 im mittleren Bereich eine Durchmesserverringerung aufweist, so dass hierdurch gleichzeitig ein Vorsprung für die Betätigung des Kraft- bzw. Lastarmes des jeweiligen Schneidekörpers gebildet wird. Der Grundkörper 27 besitzt eine seitliche Querbohrung 61, in die ein Führungskörper einbringbar ist, dessen freies Ende aus der Öffnung 42 herausragen kann. In diesem Fall kann das Organ 34 als ein Drahtstück ausgebildet sein.

Mit einer ähnlichen Querbohrung 61 ist auch das in Fig. 6 dargestellte Instrument versehen, wobei der Körper 11 so bemessen ist, dass er die Schneidekörper voll aufnehmen kann, wie es im Falle des Instruments nach Fig. 3 offenbart ist.

Die Besonderheiten der Instrumente nach den Figuren 8 bis 11 bestehen zunächst darin, dass das als Seil-, Rohr- oder Drahtstück ausgebildete Organ 32 bzw. 34 die Klemmkörper 48, 49 und 50 trägt, die abhängig von der Betriebslage der Schneidekörper 21 und 22 mit dem Kraft- bzw. Lastarm 43 und 44 des jeweiligen Schneidekörpers 21 und 22 zusammenarbeiten. Durch die relative Verstellung des Organs 32 mit Bezug auf den Grundkörper 26 werden auch die Klemmkörper 48 und 49 mit Bezug auf die Schwenkachse 36 verstellt, wobei sie auf den jeweiligen Last- bzw. Kraftarm innenseitig Druck ausüben, so dass diese nach innen bzw. aussen verschwenkt werden. Im Falle des Instruments nach Fig. 10 ist das Organ 32 ein Drahtstück. Um auch in diesem Falle den Körper 14 in das Gefäß problemlos einführen zu können, ist im stirnseitigen Endstück des Körpers 14 eine Querbohrung 60 ausgebildet, in die ein Führungsdrahtstück einbringbar ist.

Die Besonderheit des Instruments nach Fig. 11 besteht darin, dass die Klemmkörper 50 mit dem Abschnitt 30 fest verbunden sind und aus einem elastisch verformbaren Werkstoff bestehen. Daher ist es erforderlich, nur den Klemmkörper 48 zu versetzen. Ist der Klemmkörper 48 mit den Kraftarmen 43 nicht im Eingriff, dann üben die Klemmkörper 55 auf die Lastarme 44 Kraft aus, so dass sie ausgeschwenkt werden und die in Fig. 11 dargestellte Position einnehmen.

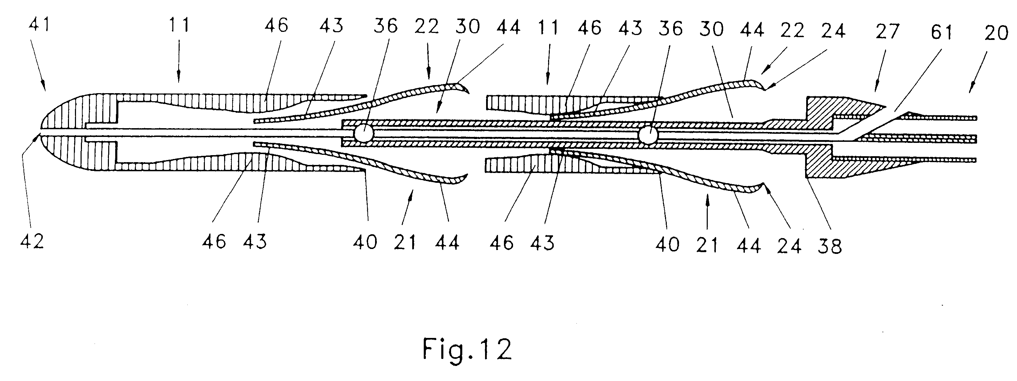

Der in Fig. 12 dargestellte Teil eines medizinischen Instruments zeichnet sich dadurch aus, dass zwei Schneidekörper-Paare 21, 22 in Serie angeordnet sind und von demselben Betätigungsmittel 20 verstellbar sind. Ganz allgemein könnten auch drei, vier oder mehrere Schneidekörper-Paare eingesetzt werden. Ganz allgemein könnten die Schneidekörper so geschaffen sein, wie es in den Ansprüchen definiert ist.

Zusammenfassend kann festgestellt werden, dass das vorgeschlagene Instrument aus im Wesentlichen folgenden Teilen besteht:

Grundkörper 26 und 27, an dem mindestens zwei Schneidekörper angelenkt sind, die durch Verstellen des Körpers bzw. der Klemmkörper sich scherenartig öffnen bzw. schließen. Im Betriebszustand sind die Schneidekanten 24 im Wesentlichen ausserhalb des Körpers angeordnet, wobei sie den Grundkörper 26 überragen und beim Betätigen des Instruments in Zugrichtung mit der Innenwand der Gefäße in Wirkverbindung bringbar sind.

## Patentansprüche

1. Medizinisches Instrument zur Beseitigung von Ablagerungen in Arterien- und/oder Venenwänden mit einem in die Gefäße einbringbaren und zumindest teilweise hohlen Körper (10 - 14) mit einem aus den Gefäßen ausführbaren Zug- und/oder Betätigungsmittel (20) sowie mit in Zugrichtung weisenden und die Ablagerungen beseitigenden Schneidekörpern (21, 22) mit Schneidekanten (24),
gekennzeichnet durch
einen in den Körper (10-14) zumindest teilweise hineinragenden Grundkörper (26,27), an dem die in Zugrichtung sich erstreckenden und als angeformte Blättchenfeder ausgebildete Schneidekörper (21, 22) angelenkt sind.

2. Instrument nach Anspruch 1,
dadurch gekennzeichnet,
dass der in den Körper (10 - 14) hineinragende Abschnitt (30) des Grundkörpers (26, 27) als ein Rohrabschnitt ausgebildet ist, in dem ein Organ (32, 34) zur relativen Verstellung des Körpers (10 - 14) mit Bezug auf den Schneidekörper (21, 22) angeordnet ist.

3. Instrument nach Anspruch 1 oder 2,
dadurch gekennzeichnet,
dass das Organ (32, 34) als ein Rohrstück oder Drahtstück ausgebildet ist, das von aussen her betätigbar ist.

4. Instrument nach einem der Ansprüche 1 bis 3,
dadurch gekennzeichnet,
dass die Schwenkachse (36) der Schneidekörper (21, 22) sich quer zur Zugrichtung des Körpers (10 - 14) erstreckt.

5. Instrument nach einem der Ansprüche 1 bis 4,
dadurch gekennzeichnet,
dass die Schneidekörper (21, 22) als ein- oder zweiarmige Hebel ausgebildet sind, die in ihrer Ruhestellung vollständig oder annähernd vollständig im Körper (10 - 14) untergebracht sind.

6. Instrument nach einem der Ansprüche 1 bis 5,
dadurch gekennzeichnet,
dass der Körper (26, 27) einen Anschlagabschnitt (38) aufweist, der in Ruhestellung der Schneidekörper (21, 22) annähernd abstandsfrei zu einer Stirnseite (40) des Körpers (10 - 14) angeordnet ist.

7. Instrument nach einem der Ansprüche 1 bis 6,
dadurch gekennzeichnet,
dass der Körper (10-14) im Wesentlichen als ein Hohlzylinder ausgebildet ist, der die Schneidekörper (21, 22) in ihrer Ruhestellung aufnimmt.

8. Instrument nach einem der Ansprüche 1 bis 7,
dadurch gekennzeichnet,
dass die vom Benutzer abgekehrte Stirnseite (41) des Körpers (10 - 14) eine in Zugrichtung sich erstreckende Öffnung (42) aufweist.

9. Instrument nach einem der Ansprüche 1 bis 8,
dadurch gekennzeichnet,
dass die vom Benutzer abgekehrte Stirnseite (41) des Körpers (10 - 14) konvex und kantfrei ist.

10. Instrument nach einem der Ansprüche 1 bis 9,
dadurch gekennzeichnet,
dass zwei Schneidekörper (21, 22) mit einer gemeinsamen Schwenkachse (36) und jeweils einem Kraft- und einem Lastarm (43, 44) vorgesehen sind.

11. Instrument nach einem der Ansprüche 1 bis 10,
dadurch gekennzeichnet,
dass die Innenwand des Körpers (10, 11, 12) Druckkörper (46) aufweist, die in ihrer Betriebsstellung mit dem jeweiligen Kraftarm (43) und in ihrer Ruhestellung mit dem jeweiligen Lastarm (44) der Schneidekörper (21, 22) in Druckverbindung stehen.

12. Instrument nach einem der Ansprüche 1 bis 11,
dadurch gekennzeichnet,
dass die Druckkörper (46) als radiale Vorsprünge mit Gleitkurven ausgebildet sind.

13. Instrument nach einem der Ansprüche 1 bis 12,
dadurch gekennzeichnet,
dass das Außenprofil des jeweiligen Schneidekörper-Längsschnittes bogenförmig ist.

14. Instrument nach einem der Ansprüche 1 bis 13,
dadurch gekennzeichnet,
dass das als ein Seil-, Rohr- oder Drahtstück ausgebildete Organ (32, 34) Klemmkörper (48, 49, 50) trägt, die abhängig von der Betriebslage der Schneidekörper (21, 22) mit dem Kraft- bzw. Lastarm (43, 44) des jeweiligen Schneidekörpers (21, 22) zusammenarbeiten.

15. Instrument nach einem der Ansprüche 1 bis 14,
dadurch gekennzeichnet,
dass die Klemmkörper (48, 49, 50) im axialen Querschnitt keilförmig ausgebildet und mit ihren verjüngten Stirnseiten einander zugekehrt sind.

16. Instrument nach einem der Ansprüche 1 bis 15,
dadurch gekennzeichnet,
dass zwei Klemmkörper (48, 49, 50) vorgesehen sind, zwischen denen die Schwenkachse der Schneidekörper (21, 22) angeordnet ist.

17. Instrument nach einem der Ansprüche 1 bis 16,
dadurch gekennzeichnet,
dass einer der Klemmkörper (50) vom Grundkörper (26) getragen ist und aus einem elastisch verformbaren Werkstoff besteht.

18. Instrument nach einem der Ansprüche 1 bis 17,
dadurch gekennzeichnet,
dass der Körper (10 - 14) und/oder der Grundkörper (26, 27) Querbohrungen (60, 61) für Führungsmittel aufweist/aufweisen.

19. Instrument nach einem der Ansprüche 1 bis 18,
dadurch gekennzeichnet,
dass die Schneidekörper (21, 22) einen als Kammer ausgebildeten Hohlraum (52) definieren, durch den sich das Organ (35) hindurch erstreckt.

20. Instrument nach einem der Ansprüche 1 bis 19,
dadurch gekennzeichnet,
dass die an die Schneidekanten (24) sich anschließenden Flanken (54, 55) der Schneidekörper (21, 22) zumindest teilweise mit Schneideabschnitten versehen sind.

21. Instrument nach einem der Ansprüche 1 bis 20,
gekennzeichnet durch
einen in den Körper (10 - 14) zumindest teilweise hineinragenden Grundkörper (26, 27), mit dem die in Zugrichtung sich erstreckenden Schneidekörper (21, 22) z.B. durch Schrauben, Schweißen, Hartlötung, Kaltverformung oder dergleichen fest verbunden sind und ihre Betriebs- bzw. Ruhestellung durch Verstellung des Körpers (10 - 14) in Zugrichtung definierbar ist.

22. Instrument nach einem der Ansprüche 1 bis 21,
dadurch gekennzeichnet,
dass die Schneidekörper (21, 22) aus einem elastisch verformbaren Werkstoff wie Metall, Keramik, Kunststoff oder aus Legierungen bestehen.

23. Instrument nach einem der Ansprüche 1 bis 22,
dadurch gekennzeichnet,
dass der Körper (10 -14), der Grundkörper (26, 27) und Teile des Schneidekörpers (21, 22) aus elektrisch leitendem Werkstoff bestehen und bis auf die scharfen Schneidekanten mit einem Isolator beschichtet sind.

24. Instrument nach einem der Ansprüche 1 bis 23,
dadurch gekennzeichnet,
dass mindestens zwei Schneidekörperpaare (21, 22) in Serie angeordnet sind.
